(19) Europäisches Patentamt
European Patent Office
Office européen des brevets

(11) Veröffentlichungsnummer: **0 423 745 A1**

# (12) EUROPÄISCHE PATENTANMELDUNG

(21) Anmeldenummer: **90119884.6**

(51) Int. Cl.5: **C07C 43/303**, C07C 41/56

(22) Anmeldetag: **17.10.90**

(30) Priorität: **20.10.89 DE 3934909**

(43) Veröffentlichungstag der Anmeldung:
**24.04.91 Patentblatt 91/17**

(84) Benannte Vertragsstaaten:
**AT BE DE FR GB IT NL SE**

(71) Anmelder: **HOECHST AKTIENGESELLSCHAFT**
**Postfach 80 03 20**
**W-6230 Frankfurt am Main 80(DE)**

(72) Erfinder: **Lachhein, Stephen, Dr.**
**Kiedricher Strasse 18**
**W-6238 Hofheim am Taunus(DE)**

## (54) Kontinuierliches Verfahren zur Herstellung von Ketalen.

(57) Verfahren zur Herstellung von Verbindungen der Formel I

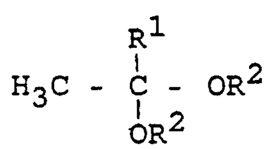

(I),

worin
$R^1$ = $C_3$, $C_4$-Alkyl und
$R^2$ = $(C_1-C_2)$Alkyl bedeuten, durch Umsetzung einer Verbindung der Formel II

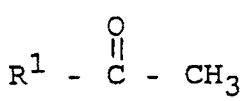

(II)

mit den Verbindungen der Formeln III und IV

$R^2 - OH$ (III) $HC(OR^2)_3$ (IV), worin $R^1$ und $R^2$ die obengenannten Bedeutungen besitzen,
dadurch gekennzeichnet, daß man eine Mischung der Ausgangsstoffe II-IV kontinuierlich an einem sauren Kontakt vorbeiströmen läßt.

EP 0 423 745 A1

## KONTINUIERLICHES VERFAHREN ZUR HERSTELLUNG VON KETALEN

Gegenstand der vorliegenden Erfindung ist ein Verfahren zur Herstellung von Verbindungen der Formel I

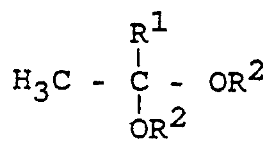
(I),

worin
$R^1$ = $C_3$, $C_4$-Alkyl und
$R^2$ = $(C_1\text{-}C_2)$Alkyl bedeuten, durch Umsetzung einer Verbindung der Formel II

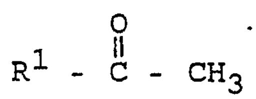
(II)

mit den Verbindungen der Formeln III und IV

$R^2 - OH$ (III)  $HC(OR^2)_3$ (IV), worin $R^1$ und $R^2$ die obengenannten Bedeutungen besitzen,

dadurch gekennzeichnet, daß man eine Mischung der Ausgangsstoffe II-IV kontinuierlich an einem sauren Kontakt vorbeiströmen läßt.

$R^1$ bedeutet bevorzugt iso-Propyl.

Die Ketale der Formel I sind wertvolle Zwischenprodukte bei der Herstellung von Substanzen, welche die phytotoxischen Nebenwirkungen von Pflanzenschutzmitteln wirksam reduzieren (Safener; Deutsche Patentenmeldungen P 38 08 896.7 und P 39 10 248.3).

Bekannt ist, daß Acetale und Ketale nach laborüblichen Verfahren durch sauer katalysierte Umsetzungen der Aldehyde bzw. Ketone mit Alkoholen und Orthoestern dargestellt werden können (Houben-Weyl, Band VI/3 119ff; C. Ferri, Reaktionen der organischen Synthese, 426-427, Georg Thieme Verlag Stuttgart 1978; Houben-Weyl, Band VI/3, 205, 221). Jedoch reagieren die Ketone nur sehr schlecht nach diesen Varianten (Houben-Weyl, Band VI/3, 204, Houben-Weyl, Band VII/1, 419).

Um mit verzweigten Aldehyden und Ketonen Ausbeuten von maximal 40-90 % zu erhalten, müssen Alkohol und/oder Orthoameisensäureester bezogen auf die Carbonylverbindung in großem Überschuß eingesetzt werden. So erhält man mit dem sterisch anspruchslosen Keton Aceton 73-75 % Ausbeute, während mit dem stärker verzweigten Pikolin lediglich noch Ausbeuten von 41 % erhalten werden (Houben-Weyl, Band VI/3, 222 und 223). Als Säuren werden dabei z.B. Schwefelsäure, para-Toluolsulfonsäure, Eisen-(III)-chlorid oder Ammoniumchlorid eingesetzt. Diese Säuren werden in katalytischen Mengen verwendet. Nach der unvollständig ablaufenden Reaktion muß das Acetal oder Ketal jedoch von den Säuren durch Neutralisation befreit werden, da anderenfalls bei der Aufreinigung der Produkte durch Destillation (Abtrennung von Nebenprodukten und Ausgangsmaterialien) entweder Zersetzung der Produkte in Enolether (Organikum, 7. Auflage 1967, Seite 221 und 222) oder die Rückspaltung zu den Carbonylverbindungen eintritt (Organikum, 7.Auflage 1967, Seite 376, 377). Die destillative Reinigung der Ketale I ist notwendig, da die anderenfalls noch vorhandenen nicht umgesetzen Carbonylverbindungen II und Alkohole III als reaktive Spezies unerwünschte Nebenreaktionen in nachfolgenden Synthesestufen eingehen können. Diese nachteilige Situation der unvollständigen Reaktion und der sich anschließenden notwendigen Reinigung der Produkt durch Destillation nach vorheriger Neutralisation der Säuren ist im technischen Maßstab aus ökologischen und ökonomischen Gründen nicht zu vertreten.

Es sind auch neuere Verfahren bekannt, bei denen als saure Katalysatoren saure Ionenaustauscher auf Kunstharzbasis wie z.B. ®Amberlyst-15 (S.A. Patwardhan und S.Dev, Synthesis 1974, 348), saure Tonerden wie z.B. Montmorillonite clay K-10 (E.C. Taylor, C.-S. Chang, Synthesis 1977, 407) oder Molekularsiebe

(D.P. Roelofsen und H. van Bekkum, Synthesis 1972, 419) eingesetzt werden, die nach beendeter Reaktion durch Filtration von der Reaktionsmischung entfernt werden können. Alle diese neueren Verfahren haben jedoch den Nachteil, daß man die sauren Katalysatoren nicht mehr "katalytisch" sondern gewichtsmäßig bezogen auf die Carbonylverbindung in nahezu gleichen Teilen einsetzt. Diese großen Katalysatormengen sind für einen großtechnischen Maßstab nicht akzeptabel, da sie entweder aufgearbeitet oder entsorgt werden müssen. Darüberhinaus werden die beschriebenen Verfahren diskontinuierlich durchgeführt, was in schlechten Raum-Zeit-Ausbeuten resultiert und große Anlagen mit Reaktionskesseln und Destillationskolonnen erfordert.

Alle diese geschilderten Nachteile vermeidet das erfindungsgemäße Verfahren, bei dem die Ketale der Formel I in nahezu quantitativen Ausbeuten (> 98 %) und in so hohen Reinheiten erhalten werden, daß diese Produkte ohne zusätzliche Reinigungsoperationen direkt in Folgeumsetzungen eingehen können. Zudem ist das Verfahren aufgrund seiner kontinuierlichen Fahrweise verfahrenstechnisch besonders einfach zu handhaben.

Das erfindungsgemäße Verfahren wird so durchgeführt, daß man eine Mischung der Ausgangsstoffe II-IV kontinuierlich an einem sauren, evtl. vorgewärmten Kontakt vorbeiströmen läßt. Der saure Kontakt befindet sich zweckmäßigerweise in einem Strömungsrohr wie es von Ionenaustauschersäulen bekannt ist oder in einer dem Fachmann bekannten Umpumpapparatur, die mit einem Überlauf ausgestattet sein kann.

Als saurer Kontakt dienen saure Ionenaustauscher. Neben den obengenannten sauren Tonerden und Molekularsieben sind insbesondere saure Ionenaustauscher auf Kunstharzbasis für das erfindungsgemäße Verfahren von Bedeutung. Diese sind in einer breiten Palette im Handel erhältlich. Beispielshaft seien die ®Amberlite und ®Amberlyst-Typen der Firma Rohm & Haas genannt.

Für 1 Mol Keton der Formel I werden ca. 2-3 g an saurem Kontakt und somit lediglich katalytische Mengen benötigt.

Die Temperatur (Reaktionstemperatur), bei der die Mischung aus den Substanzen der Formeln II, III und IV an dem sauren Kontakt vorbeiströmt, liegt zwischen 20 °C und 100 °C, vorzugsweise zwischen 30 °C und 80 °C.

Die Ausgangstoffe II-IV werden in der Regel in äquimolarem Verhältnis eingesetzt. Der Orthoester IV und der Alkohol III können auch im Überschuß (bis ca. 1,5 fach) bezogen auf das Keton II vorliegen. Auch kann der Alkohol oder der Orthoester in einem deutlichen Unterschuß gegenüber dem Keton verwendet werden.

Das erfindungsgemäße kontinuierliche Verfahren ist als um so überraschender zu bewerten, da die Durchflußzeit und damit der direkte Kontakt der Mischung mit dem sauren Kontakt (Katalysator) im Mittel bei maximal 20 min liegt. Die Durchflußzeit ermittelt sich aus der durchschnittlichen Verweilzeit der Reaktionsmischung im Strömungsrohr bzw. in der Umpumapperatur.

Aus der Literatur ist bekannt, daß bei der Reaktion der Ketone II mit den Verbindungen III und IV in Gegenwart von sauren Ionenaustauschern Reaktionszeiten von 3 bis 40 Stunden zur Erzielung von befriedigenden Ausbeuten benötigt werden. Dabei wird mit drastischen Überschüssen an den Edukten III und/oder IV und großen Mengen an saurem Ionenaustauscher gearbeitet (E.C. Taylor, C.S. Chang, Synthesis 1977, 467).

Die nachfolgenden Beispiele dienen zur Erläuterung der Erfindung:

**Beispiel 1**

2,2-Dimethoxy-3-methylbutan

Ein Strömungsrohr vom Durchmesser 1,5 cm wird mit 10 g saurem Ionenaustauscher ®Amberlyst A-16 (Firma Rohm & Haas) gefüllt (Gesamtfüllhöhe 10 cm). Dieser Ionenaustauscher wird auf 50 °C erwärmt und während 4 h wird eine Mischung, bestehend aus 866 g (10,0 Mol) Methylisopropylketon, 1121 g (10,5 Mol) Orthoameisensäuretrimethylester und 323,2 g (10,0 Mol) Methanol am Ionenaustauscher vorbeiströmen lassen. Die Reaktionstemperatur liegt zwischen 50 und 70 °C. Es wurden innerhalb von 4 Stunden 2282,4 g Strömungsfiltrat erhalten, das nach Abstreifen der Leichtsieder Ameisensäuremethylester und Methanol am Dünnschichtverdampfer zu 1325 g Produkt 2,2-Dimethoxy-3-methylbutan von einer Reinheit von 98,4 % führt, was einer Ausbeute von 98,7 % d.Th. entspricht.

Die Kontaktzeit der Mischung mit dem sauren Ionenaustauscher ergibt sich im Mittel zu 2,1 min und wird wie folgt berechnet.
Volumen vom Ionenaustauscher in der Säule = 23,5 $cm^3$.

Volumen der Reaktionsmischung 2640 $cm^3$, bestehend aus 866 g Methylisopropylketon, 323 g Methanol und 1121 g Orthoameisensäuretrimethylester. Diese 2640 $cm^3$ fließen während 240 min an dem Kontakt von 23,5 $cm^3$ Volumen vorbei:

$$\frac{2640\ cm^3}{23{,}5\ cm^3} = 112{,}3 \qquad \frac{240\ min}{112{,}3} = 2{,}1\ min \qquad \text{Kontaktzeit}$$

**Beispiel 2**

2,2-Dimethoxy-3-methylbutan

In einer Umpumpapparatur wird innerhalb von 4 Stunden eine Mischung bestehend aus 866 g (10,0 Mol) Methylisopropylketon, 1121 g (10,5 Mol) Orthoameisensäuretrimethylester und 323,2 g (10,0 Mol) Methanol an dem sauren Ionenaustauscher ®Amberlyst A-15 vorbeigepumpt, wobei durch den Überlauf während dieser Zeit 2360 g Lösung ablaufen können. Die Umpumpapparatur hat einen Durchmesser von 1,5 cm und die Füllhöhe des Ionenaustauschers beträgt 10 cm. die Umpumpapparatur wurde zu Beginn der Reaktion mit Methanol gefüllt, damit ein sofortiger Überlauf eintreten kann. Am Ende der Reaktion wurde mit Methanol nachgefahren, damit alles Wertprodukt aus der Umpumpapparatur herausgespült wird. Bei größeren Ansätzen und über eine längere Reaktionszeit erübrigt sich diese Maßnahme, die hier lediglich zur genauen Ausbeutebestimmung dient. Die Reaktionstemperatur entspricht der aus Beispiel 1. Ein Vorwärmen der Reaktionsmischung ist möglich aber nicht notwendig.

Nach Abstreifen am Dünnschichtverdampfer von den Leichtsiedern Ameisensäuremethylester und Methanol werden 1321 g Produkt erhalten. Dieses Produkt hat eine Reinheit von 98.3 %, was einer Ausbeute von 98,4 % d.Th. entspricht.

Die Kontaktzeit der Mischung mit dem sauren Ionenaustauscher ergibt sich im Mittel ebenfalls zu 2,1 min.

**Ansprüche**

1. Verfahren zur Herstellung von Verbindungen der Formel I

$$H_3C - \overset{R^1}{\underset{OR^2}{C}} - OR^2 \qquad (I),$$

worin

$R^1$ = $C_3$,$C_4$-Alkyl und

$R^2$ = $(C_1$-$C_2)$Alkyl bedeuten, durch Umsetzung einer Verbindung der Formel II

$$R^1 - \overset{O}{\overset{\|}{C}} - CH_3 \qquad (II)$$

mit den Verbindungen der Formeln III und IV

$$\underset{(III)}{R^2 - OH} \qquad \underset{(IV)}{HC(OR^2)_3} \quad \text{, worin } R^1 \text{ und}$$

$R^2$ die obengenannten Bedeutungen besitzen,

dadurch gekennzeichnet, daß man eine Mischung der Ausgangsstoffe II-IV kontinuierlich an einem sauren Kontakt vorbeiströmen läßt.

2. Verfahren gemäß Anspruch 1, dadurch gekennzeichnet, daß $R^1$ iso-Propyl bedeutet.

3. Verfahren gemäß Anspruch 1 oder 2, dadurch gekennzeichnet, daß als saurer Kontakt ein saurer Ionenaustauscher auf Kunstharzbasis verwendet wird.

4. Verfahren gemäß einem oder mehreren der Ansprüche 1 bis 3, dadurch gekennzeichnet, daß die Reaktionstemperatur 20 °C - 100 °C beträgt.

5. Verfahren gemäß einem oder mehreren der Ansprüche 1 bis 4, dadurch gekennzeichnet, daß die Reaktionstemperatur 30 °C - 80 °C beträgt.

Europäisches
Patentamt

# EUROPÄISCHER RECHERCHENBERICHT

Nummer der Anmeldung

**EP 90 11 9884**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | SYNTHESIS, INTERNATIONAL JOURNAL OF METHODS IN SYNTHETIC ORGANIC CHEMISTRY, Nr. 5, Mai 1974, Seiten 348-349; S.A. PATWARDHAN et al.: "Amberlyst-15, a superior catalyst for the preparation of enol ethers and acetals" * Ganzes Dokument * | 1-5 | C 07 C 43/303<br>C 07 C 41/56 |
| D,A | SYNTHESIS, INTERNATIONAL JOURNAL OF METHODS IN SYNTHETIC ORGANIC CHEMISTRY, Nr. 7, Juli 1977, Seite 467; E.C. TAYLOR et al.: "Trimethyl orthoformate absorbed on the montmorillonite clay K-10; an effective reagent for acetal formation" * Ganzes Dokument * | 1-5 | |
| A | US-A-2 566 559 (A.A. DOLNICK et al.) * Anspruch 1 * | 1-5 | |
| A | DE-A-2 929 827 (BASF) * Anspruch 1 * | 1-5 | |
| | | | **RECHERCHIERTE SACHGEBIETE (Int. Cl.5)** |
| | | | C 07 C 41/00<br>C 07 C 43/00 |

Der vorliegende Recherchenbericht wurde für alle Patentansprüche erstellt

| Recherchenort | Abschlußdatum der Recherche | Prüfer |
|---|---|---|
| Den Haag | 04 Januar 91 | ZERVAS B. |

KATEGORIE DER GENANNTEN DOKUMENTE
X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument